# EUROPEAN PATENT APPLICATION

(11) **EP 0 717 994 A1**
(43) Date of publication of application: **26.06.1996**
(21) Application number: 94919682.8
(22) Date of filing: 07.07.1994
(51) Int. Cl.: A61K 31/57

(54) **NEW FORMULATION FOR THE TOPICAL TREATMENT OF PSORIASIS**

(71) Applicant: Lujambio Galdeano, Ignacio Anton, E-20009 San Sebastián (ES)
(72) Inventor: Lujambio Galdeano, Ignacio Anton, E-20009 San Sebastián (ES)
(74) Representative: Richebourg, Michel François
(86) International application number: ES9400070
(87) International publication number: WO9601633

(57) **Abstract**

New formulation for the topical treatment of psoriasis based on betametasone dipropionate, retinoic acid and urea.

## Description

Psoriasis is a skin ailment generally requiring medical treatment. Treatment of any ailment with medicinal components always entails the danger of producing possible undesirable secondary effects due to which the precepts recommend the use of the most specific ingredients, in other words those best suited to the purpose without upsetting other functions involved in the good performance of other parts of the body.

The ideal treatment of all skin ailments is external, which means that only the affected area is subjected to the appropriate medication.

The MEDICINE FOR THE EXTERNAL TREATMENT OF PSORIASIS bears these simple yet nevertheless important aspects in mind in providing the desired cure.

The curative advantage that the MEDICINE FOR THE EXTERNAL TREATMENT OF PSORIASIS offers is achieved through the synergy of the components in the formula, with this synergy eliminating the ailment in a spectacular way. The actual proportion between the ingredients is an issue which admits a certain variability, in such a way that the following composition gives an illustrative, but not limitative, example of those proportions:

| | |
|---|---|
| Betametasone Dipropionate | 0.5% |
| Retinoic acid | 0.05% |
| Urea | 10% |
| Excipient | c.s. |

The nature of the excipient will determine the nature of the galenic preparation, and it can be any of those that are used by the pharmaceutical profession for external treatments such as, for example, ointment.

## Claims

1. MEDICINE FOR THE EXTERNAL TREATMENT OF PSORIASIS characterized by it being a mixture of components in which the active ingredients of dipropionate of betametasone, retinoic acid, and urea are found present simultaneously.

2. MEDICINE FOR THE EXTERNAL TREATMENT OF PSORIASIS in accordance with the previous claim, characterized by its being suitable for application in any of the galenic preparations used by the pharmaceutical profession for external treatments.
